# EUROPEAN PATENT APPLICATION

(11) **EP 2 826 497 A2**
(43) Date of publication of application: **21.01.2015**
(21) Application number: 14176061.1
(22) Date of filing: 08.07.2014
(51) Int. Cl.: A61L 27/50, A61L 27/54, A61L 27/20

(54) **Intra-articular injection and implant attachment of molecules consisting of lubricin and extracellular superoxide dismutase or hyaluronic acid and extracellular superoxide dismutase**

(30) Priority: 08.07.2013 US 201361843666 P
(71) Applicant: Arthrex, Inc., Naples, FL 34108-1945 (US)
(72) Inventor: Roller, Brandon L., Naples, FL Florida 34109 (US); Bare, Christopher M., Naples, FL Florida 34117 (US); Dutta Roy, Tithi, Unit 104 Estero, FL Florida 33928 (US); Albertorio, Ricardo, Naples, FL Florida 34110 (US)
(74) Representative: Leihkauf, Steffen Falk

(57) **Abstract**

Formulations, mixtures, compositions and techniques for improved treatment options for osteoarthritis and intra-articular joint degeneration. The formulations and compositions are provided in the form of injectable aqueous formulations that increase the delivery of a lubricating agent (lubrication molecules) into the joint to resist breakdown, decrease free radical production (by neutralizing these radicals), and increase removal of superoxide byproducts in the synovial fluid. The injectable aqueous formulations are preferably administered into the intra-articular space of an intra-articular joint. The lubricating formulation includes molecules of lubricin and SOD, hyaluronic acid and SOD, or lubricin and SOD and hyaluronic acid, or hyaluronic acid and lubricin, or Vitamin E and SOD or hyaluronic acid, or combinations thereof.

## Description

### FIELD OF THE INVENTION

The present invention relates to improved technologies for osteoarthritic repairs.

### BACKGROUND OF THE INVENTION

Osteoarthritis (OA) is a debilitating joint disease that affects nearly thirty million people in the United States. Osteoarthritis results in progressive and irreversible degenerative changes in synovial joints, starting with the articular cartilage, then attacking the structure of the articulating bones and finally affecting all components of the joint. Osteoarthritis involves free radical production which leads to pathological changes within the joint. Both the viscosity and the quantity of synovial fluid are reduced in osteoarthritis. This increases friction between the sliding components of the joint and reduces their ability to cushion axial forces, leading to further joint damage.

Hyaluronic acid is a major component of synovial fluid and determines its ability to lubricate the joint. In addition to its viscoelastic properties, pre-clinical and clinical studies have reported that hyaluronic acid also has chondro-protective and antiinflammatory properties in the joint. Low-molecular weight hyaluronic acid and high-molecular weight hyaluronic acid have been used for intra-articular treatment for osteoarthritis in injection form. One of the treatments involves sorbitol which is attached to a hyaluronic acid chain, to prevent free radical breakdown of the hyaluronic acid.

Extracellular superoxide dismutase [Cu-Zn] ("EC-SOD" or "SOD3") is a free radical scavenger that has been shown to be decreased during the progression of osteoarthritis.

Lubricin is another type of lubrication molecule that is found in reduced concentrations during the osteoarthritic progression.

There is a need for formulations, compositions, mixtures and methods of binding SOD (such as SOD3) to a hyaluronic acid molecule or binding SOD to a lubricin molecule, or a mixture of both. Also needed are methods of administering such formulations, compositions, mixtures to allow for the delivery of the lubricating mixture (agent or lubrication molecule) into a joint that has the potential of resisting breakdown and removing excess superoxide byproducts.

Also needed are methods of binding SOD (such as SOD3) to implants, or for coating implants with SOD, or attaching SOD to implants, or incorporating SOD into implants. Methods of coating implants with a lubrication molecule to which SOD is attached to (i.e., having SOD3 attached to it) are also needed, as well as methods in the treatment and/or prevention of pain associated with osteoarthritis and intra-articular joint degeneration.

### BRIEF SUMMARY OF THE INVENTION

The present invention provides mixtures, formulations, compositions and techniques for improved treatment options for osteoarthritis and intra-articular joint degeneration. The invention provides methods and compositions in the form of injectable aqueous formulations that increase the delivery of a lubricating agent (lubrication molecules) into the joint to resist breakdown, decrease free radical production (by neutralizing these radicals), and increase removal of superoxide byproducts in the synovial fluid. The injectable aqueous formulations are preferably administered directly into the intra-articular space of an intra-articular joint.

The lubricating formulation includes molecules of lubricin and SOD, hyaluronic acid and SOD, or lubricin and SOD and hyaluronic acid, or hyaluronic acid and lubricin, or any combinations thereof. The molecules may be bound or individualized. In exemplary embodiments only, the lubricating agent consists of bound molecules of lubricin and SOD, hyaluronic acid and SOD, or lubricin and SOD and hyaluronic acid, or hyaluronic acid and lubricin, or combinations thereof. In additional embodiments, Vitamin E may be added in any or all of the combinations.

In exemplary-only embodiments, the present invention provides intra-articular application (by direct intra-articular injection, for example) of (1) a hyaluronic acid molecule with bound lubricin molecules; (2) a hyaluronic acid molecule with bound SOD molecules; (3) a hyaluronic acid molecule with bound lubricin and SOD molecules; (4) a lubricin molecule with bound SOD; and (5) a lubrication molecule with bound SOD. In exemplary embodiments, SOD is SOD3.

The present invention also provides methods of (6) binding SOD to implants such as UHMWPE; (7) binding SOD to degradable implants (e.g., polylactic or polyglycolic acid, composites of ceramic/polymer, etc.); (8) coating or binding or incorporating molecules that comprise a hyaluronic acid molecule with bound SOD molecules to implants such as UHMWPE; (9) coating or binding molecules that comprise a hyaluronic acid molecule with bound lubricin and SOD molecules to/into implants such as UHMWPE; (10) coating or binding or incorporating molecules that comprise a lubricin molecule with bound SOD to/into implants such as UHMWPE; and (11) coating or binding or incorporating molecules that comprise lubrication molecule(s) with bound SOD to/into implants such as UHMWPE. In exemplary embodiments, SOD is SOD3.

The lubricating formulations may optionally comprise blood products, for example, autologous or allogenic blood product such as plasma, autologous conditioned plasma, platelet-rich plasma, lyophilized platelets, bone marrow, bone marrow aspirate, bone marrow concentrate, stem cells such as concentrated or expanded stem cells (derived from a variety of sources), or any combinations of these products. The lubricating formulations may optionally include Vitamin E.

The present invention also provides methods of adding, binding, incorporating or compounding SOD with degradable implants such as PLA and/or composite implants. SOD (particularly, SOD3) could be mixed with the polymer or ceramic, then molded or extruded/machined into different fixation devices and implants such as anchors, screws or pins, among many others. The SOD would be mixed throughout the fixation device (implant) offering *in vivo* exposure at implantation and throughout the degradation profile.

Other features and advantages of the present invention will become apparent from the following description of the invention.

### DETAILED DESCRIPTION OF THE INVENTION

The present invention provides techniques and methods of using SOD, and particularly SOD3, bound to at least one lubricating molecule (alone or in combination with additional molecules/components). The invention also provides using SOD for binding it to a hyaluronic acid molecule, or binding it to a lubricin molecule, or binding it to Vitamin E, or binding it to any of the combined components and mixtures thereof, that would allow for the delivery of the lubricating agent (lubricating molecules) into a joint that has the potential of resisting breakdown and removing excess superoxide byproducts.

An additional use for SOD is binding this molecule to implants (non-degradable implants such as UHMWPE or degradable implants such as PLA, PGA, or composite implants) or coating implants with this molecule, or coating implants with a lubrication molecule that SOD is attached to, or incorporating these molecules into the implant material before or during the fabrication of the implant. It is known that free radicals are produced when UHMWPE is irradiated. These radicals then cause oxidative degradation in the material. The addition of a superoxide dismutase to stabilize UHMWPE provides a more effective way of neutralizing these radicals.

Although the embodiments below will be described with particular reference to SOD3, it must be understood that the invention is not limited to these exemplary-only embodiments and contemplates other forms of SOD (other SOD isozymes) known in the art.

The inventors of the present invention have shown that two particular proteins (lubricin and SOD3) are deficient after meniscal/ACL/osteoarthritis pathology (as detailed in Studies A and B below). Study A provides details on which superoxide dismutases are decreased within pathologic meniscal tissue. Study B provides data demonstrating how lubricin and SOD3 are decreased within synovial joint fluid associated with meniscal pathology, ACL pathology, and osteoarthritis.

### Study A

The objective of Study A was to use microarray and LC-MS/MS to assess the transcriptome and proteome of normal and pathologic meniscal tissue.

### Methods

Meniscal Tissue Collection: Meniscal tissue was collected from the knees of five patient groups (n=3/group) - age range in parentheses: (1) Aged-normal (AN) (64-78): no previous injury, operative procedures, or gross meniscal/articular damage; (2) Young meniscal debridement (YMD) (28-36): minimal articular cartilage damage; (3) Older meniscal debridement (OMD) (53-57): chronic degenerative tears, moderate articular cartilage damage; (4) Mild radiographic OA, received a total knee arthroplasty (TKA) (MOA) (44-61); (5) Severe radiographic OA, received TKA (SOA) (50-69). All tissue was collected from white/white and white/red zones of posterior medial menisci and placed in RNA later.

Tissue Extraction: Total RNA was extracted from the tissue using the TriSpin method. Protein was extracted from the lower organic phase of the Trizol solution after the upper aqueous phase was removed for RNA.

Microarray Analysis: RNA from each patient was amplified, biotin labeled, and fragmented using the MessageAMP RNA amplification kit. The fragmented aRNA of each patient was pooled based on clinical group, and each pool was hybridized to a GeneChip Human Genome U133 Plus 2.0 Array (n=1/group). Expression levels were compared between the AN and clinical groups to identify genes with at least a 1.5x fold change in expression level.

Proteomics Analysis: Extracted protein from each sample was pooled based on clinical group, and 1D-PAGE was performed on 25µg of the protein pool. The five lanes were cut into 8 equal sections, trypsin digested, and each digest analyzed twice (technical replicates) by LCMS/MS using LTQ Orbitrap instrumentation at the university proteomics center. Results were evaluated using the Scaffold 2 Viewer Proteome software.

Microarray Analysis: The microarray identified 70 genes that had at least a 1.5x fold change in expression between the AN and pathologic groups. Representative genes listed are associated with extracellular and intracellular matrix (ECM, ICM) synthesis, vascularity, tissue signaling, and apoptosis.

Proteomics Analysis: Proteomics analysis identified 173 unique proteins in the meniscal tissue. Proteins were marked for further evaluation based on fold difference, spectral count, potential *in vivo* function, and correlation with microarray data. The proteins involved are: thrombospondin (cell-to-matrix interactions); transthyretin (thyroid hormone transport); anti-oxidation (extracellular superoxide dismutase); and ribonuclease (mRNA cleavage).

Study A characterizes the proteome and transcriptome of normal and pathologic meniscal tissue using microarray and LC-MS/MS on the same tissue sample. The data from this study indicate an array of pathological processes that occur during the etiopathogenesis and progression of meniscal disease. Pathologic tissue showed increased expression and synthesis of ECM/ICM and vascularity related constituents at the gene and protein levels; both indicative of attempts at tissue repair. Further, the increase in thyroid hormone signaling indicates another potential component of this reparative attempt. However, gene expression data also indicated increased apoptosis in the pathologic tissues. Increased release of reactive oxygen species from apoptotic cells coupled with the decrease in tissue EC-SOD (SOD3) noted suggests that an important mechanism of disease involves increased oxidative stress in pathologic meniscal tissues.

### Study B

The objective of Study B was to comprehensively assess meniscal pathology as it relates to synovial fluid.

### Methods

Synovial Fluid Collection: Synovial fluid (SF) was aspirated from the knee of four patient groups (n=3/group) - age range in parentheses: (1) Normal (23-28): no previous injury, operative procedures, or clinical symptoms of pain; (2) Meniscal repair (MR) (30-37): repairable tears involving vascular region of medial meniscus; (3) Young meniscal debridement (YMD) (25-33): tears involving the avascular region of medial meniscus and minimal articular cartilage damage (ICRS score of 0 or 1); (4) Older meniscal debridement (OMD) (53-56): chronic degenerative tears in avascular region of medial meniscus and moderate articular cartilage damage (ICRS score of 3).

Matrix Metalloproteinase (MMP) and Cytokine Analysis: Hyaluronidase treated SF samples were analyzed using the Fluorokine MAP human MMP (MMP-1, -2, -9, and 013) and cytokine (Interleukin 1β (IL-1β), IL-6, IL-8, Tumor necrosis factor- α (TNF-α), Macrophage inflammatory protein 1 α (MIP-1α), MIP-1β, Monocyte chemotactic protein 1 (MCP-1), RANTES) multiplex panels (R&D Systems). A log transformation was performed to normalize the data for statistical analysis. Results were evaluated using the unpaired t-test with significance set at p<0.05.

Proteomics Analysis: Two SF samples per group were hyaluronidase treated, albumin/IgG depleted, concentrated with a 3 kDa filter, and 50µg of protein separated by 1D-PAGE. Each lane was cut into 12 equal sections then trypsin digested, and each digest was analyzed by LC-MS/MS using LTQ Orbitrap instrumentation. Results were statistically evaluated with the unpaired t-test using the Scaffold 2 Viewer Proteome software with significance set at p<0.05.

MMP/Cytokine Analysis: The concentration of MMP-1 in the normal group was significantly lower than all three pathologic groups. The concentration of MMP-2 in the normal group was significantly lower than the MR and YMD groups, but not the OMD group. The concentration of MIP-1β in the normal group was significantly lower than the OMD group. IL-8 was notably, but not significantly, higher in the OMD group compared to the normal group. MMP-9, MMP-13, IL-1β, TNF-1α, and MIP-1α were below the detection limits of this assay for all patients.

Proteomics Analysis: Proteomics analysis identified 235 unique proteins in the synovial fluid samples. When the three meniscal pathology groups were combined and compared to the normal group, numerous proteins were found to be significantly different. Of these proteins, 11 of the identified proteins that were significantly higher in the pathology group and 18 that were significantly higher in the normal group were selected for further study based on fold difference, spectral count, and potential *in vivo* function. When the three meniscal pathology groups were compared separately to the normal group, many but not all, of the same proteins were significantly different between the normal group and the individual pathology groups. Very few proteins were found to be significantly different between the different pathological groups. The key proteins are as follows: alfa 2 Macroglobulin, Haptoglobin, Apolipoprotein B-100, Proteoglycan 4, Serpin Peptidase Inhibitor Clade F, Extracellular Superoxide Dismutase, Insulin-like Growth Factor Binding Protein 6.

These data provide novel information for the investigation of synovial fluid biomarkers for meniscal pathology. Importantly, the MMP and cytokine results suggest that MMP-1 may play a direct role in meniscal pathology of various types. Also, the study revealed that MIP-1β and IL-8 were only increased for the OMD group. These biomarkers have been reported to be increased in OA patients, and the OMD patients had significant cartilage damage (early OA). These two biomarkers function as indicators of early OA.

Study B also creates and optimizes a protocol for isolation of synovial fluid proteins when performing LC-MS/MS. Proteomics analysis of synovial fluid from patients with meniscal pathology has not been performed using the protocol developed for this study. This procedure allowed for improved detection and analysis of the synovial fluid proteome, and identified numerous differences between the normal and meniscal pathology groups.

Based on the above-noted findings, Applicants of the present invention have discovered that, as lubricin and SOD (particularly, SOD3) are reduced during intra-articular pathology, it would be beneficial to combine the two proteins as an injection, i.e., in the form of an injectable aqueous formulation to be administered into the intra-articular space of an intra-articular joint.

Applicants have also discovered the following formulations/mixtures/compositions for intra-articular administration preferably by injection:
1. Intra-articular injection that comprises a hyaluronic acid molecule with bound lubricin molecules.
2. Intra-articular injection that comprises a hyaluronic acid molecule with bound SOD3 molecules.
3. Intra-articular injection that comprises a hyaluronic acid molecule with bound lubricin and SOD3 molecules.
4. Intra-articular injection that comprises a lubricin molecule with bound SOD3.
5. Intra-articular injection that comprises a lubrication molecule with bound SOD3.
6. Binding SOD3 to non-degradable implants such as UHMWPE or degradable implants like PLA, PGA, and/or composites of ceramic/polymer.
7. Coating or binding a molecule that comprises a hyaluronic acid molecule with bound SOD3 molecules to implants such as UHMWPE.
8. Coating or binding a molecule that comprises a hyaluronic acid molecule with bound lubricin and SOD3 molecules to implants such as UHMWPE.
9. Coating or binding a molecule that comprises a lubricin molecule with bound SOD3 to implants such as UHMWPE.
10. Coating or binding a molecule that comprises a lubrication molecule with bound SOD3 to implants such as UHMWPE.
   Applicants have further discovered the following formulations (mixtures/compositions) for intra-articular administration preferably by injection:
11. Intra-articular injection that comprises a hyaluronic acid molecule with bound lubricin molecules combined with an autologous blood product (i.e., bone marrow, bone marrow aspirate, serum, autologous fluids such as ACP, PRP, BMA, BMC, etc.).
12. Intra-articular injection that comprises a hyaluronic acid molecule with bound SOD molecules combined with an autologous blood product (i.e., bone marrow, bone marrow aspirate, serum, autologous fluids such as ACP, PRP, BMA, BMC, etc.).
13. Intra-articular injection that comprises a hyaluronic acid molecule with bound SOD and lubricin molecules combined with an autologous blood product (i.e., bone marrow, bone marrow aspirate, serum, autologous fluids such as ACP, PRP, BMA, BMC, etc.).
14. Intra-articular injection that comprises a lubricin molecule with bound SOD molecules combined with an autologous blood product (i.e., bone marrow, bone marrow aspirate, serum, autologous fluids such as ACP, PRP, BMA, BMC, etc.).
15. Intra-articular injection that comprises an allogenic lubricin molecule combined with an allogenic blood product (i.e., PRP, lyophilized platelets, bone marrow, bone marrow aspirate, serum, etc.).
16. Intra-articular injection that comprises an allogenic lubricin molecule and allogenic SOD molecule combined with an allogenic blood product (i.e., PRP, lyophilized platelets, bone marrow, bone marrow aspirate, serum, etc.).
17. Intra-articular injection that comprises an allogenic hyaluronic acid molecule and allogenic SOD molecule combined with an allogenic blood product (i.e., PRP, lyophilized platelets, bone marrow, bone marrow aspirate, serum, etc.).
18. Intra-articular injection that comprises an allogenic hyaluronic acid molecule and allogenic SOD molecule and allogenic lubricin molecule combined with an allogenic blood product (i.e., PRP, lyophilized platelets, bone marrow, bone marrow aspirate, serum, etc.).
19. Intra-articular injection that comprises an allogenic SOD molecule combined with an allogenic blood product (i.e., PRP, lyophilized platelets, bone marrow, bone marrow aspirate, serum, etc.).
20. Intra-articular injection that comprises an allogenic hyaluronic acid molecule and allogenic lubricin molecule combined with an allogenic blood product (i.e., PRP, lyophilized platelets, bone marrow, bone marrow aspirate, serum, etc.).

Applicants provide below additional information on the two proteins (lubricin and SOD3), hyaluronic acid, as well as other similar constituents with applicability to the present invention (and particularly on how they affect (either alone or in combination) meniscal pathology as it relates to synovial fluid):

### Hyaluronic Acid

Intra-articular application of hyaluronic acid has been a recognized treatment option for osteoarthritis of the knee. Biochemical studies have shown that free radicals have destructive effects on tissues and stimulate inflammatory processes. In the joint, these substances convert hyaluronic acid directly to oligosaccharides, leading to a reduction in viscosity and molecular weight of the synovial fluid.

Sorbitol is known to be an effective scavenger of free radicals. In combination with hyaluronic acid, sorbitol acts at two levels. First, sorbitol protects hyaluronic acid from direct attack by free radicals, so that hyaluronic acid stays intact longer as an active agent. Second, reducing the concentration of free radicals decreases the migration of macrophages into the synovial membrane, resulting in reduced inflammation and less pain.

### Extracellular Superoxide Dismutase [Cu-Zn] (SOD3)

Physiologically, there is a balance between the production of reactive oxygen species (ROS) through cellular metabolism and the level of endogenous antioxidants needed to protect tissues from oxidative damage. When the production of ROS increases and/or the level of antioxidants decrease, the resulting imbalance will cause oxidative stress and potential pathophysiologic damage. The process of forming ROS begins with a one-electron reduction of oxygen to form a superoxide (O₂⁻), which can go through dismutation to hydrogen peroxide (H₂O₂) or react with nitric oxide (NO) producing a toxic product called peroxynitrite (ONOO). The breakdown product of peroxynitrite can create a highly toxic hydroxyl radical (OH). Superoxide dismutases are antioxidants that catalyze the dismutation of two superoxide radicals into oxygen and hydrogen peroxide.

Three SOD isozymes have been described, the third being termed extracellular superoxide dismutase (SOD3). SOD3 is known as being the predominant SOD in plasma, synovial fluid, and other extracellular fluids. The primary location of SOD3 (99%) is in the extravascular space of tissues reversibly bound to heparin sulphate proteoglycan ligands on the surface of cells and within the interstitial matrix. Expression of SOD3 has been shown to be decreased by inflammatory proteins such as tumor necrosis factor-α.

Superoxide radicals are capable of degrading hyaluronic acid within synovial fluid, but superoxide dismutases have been shown to protect its degradation *in vitro.* Collagens are also known targets for oxygen free radicals and will be cleaved into small peptides, but SOD3 has the ability to bind to the heparin-binding region of collagen and protect it from oxidative fragmentation. Oxygen-derived free radical degradation of nasal cartilage can be inhibited by the presence of superoxide dismutase. It has been identified that osteoarthritic cartilage will have increased levels of SOD3 mRNA but will have significantly decreased SOD3 protein levels when compared to controls. Due to this phenomenon, it has been postulated that oxidative stress will elicit an initial period of compensation, but an inadequate production of SOD3 and loss of tissue binding sites eventually prevent the ability of SOD3 to adequately counteract the increased amount of ROS that is involved in the pathophysiology of osteoarthritis.

Intra-articular injection of SOD derived from bovine erythrocytes has been used to treat osteoarthritis in human clinical studies with significant success when compared to a placebo control. SOD3 specifically has been used to treat collagen-induced arthritis in a murine model. The mice treated with SOD3 had suppression of joint swelling and deformity. Histologically, the mice had less destruction of cartilage and bone, less infiltration of mononuclear cells, and less proliferation of synovial cells. This study demonstrated how there is an inadequate amount of SOD3 to account for the oxidative stresses that occur with intra-articular phatological events. From the data above, Applicants have concluded that, when mensical pathology, ACL pathology, or degenerative joint disease occurs, the intra-articular concentration of SOD3 will be reduced when compared to normal patients.

Applicants have discovered that this protein (alone or in combination with other proteins such as HA or lubricin) offers a treatment modality for intra-articular injuries.

### Lubricin

Proteoglycan 4 (lubricin) is found within synovial fluid but it is also found along the articular surface within the superficial zone of cartilage and along the femoral and tibial surface of the meniscus along the radial and circumferential fibers. This molecule is associated with the normal lubrication of the joint and has a protective feature due to the fact that it can dissipate strain energy. Lubricin production comes from chondrocytes, synovial cells, and meniscal cells. This lubricating glycoprotein has been shown to be reduced by the progression of osteoarthritis. Lubricin has also been shown to be statistically reduced during the early stages following ACL injury when compared to the contralateral uninjured knee.

The formulations of the present invention in the form of injectable solutions have particular applicability to intra-articular joints such as the knee, hand, ankle, hip, wrist, spine, shoulder, and any other joint or articular space. SOD3 of the injectable formulations binds specifically to type I collagen through the heparin-binding region of the articular cartilage of the joint and protects it from oxidative fragmentation. The therapies of the present invention provide a beneficial effect on pain and prevention of intra-articular joint degeneration.

The injectable solutions/formulations of the present invention may optionally comprise additional components such as proteins, growth factors or chemicals that may be provided within the solutions/formulations.

In accordance with exemplary-only embodiments, the injectable solutions/formulations may be obtained to additionally comprise components such as growth factors, additional antiseptic chemicals and/or antibiotics and/or electrolytes, and/or hormones or site-specific hybrid proteins (that promote or enhance the wound healing effectiveness of the growth factors), and/or autologous or allogenic blood products such as plasma, autologous conditioned plasma, platelet-rich plasma, lyophilized platelets, bone marrow, bone marrow aspirate, bone marrow concentrate, stem cells such as concentrated or expanded stem cells (derived from a variety of sources), serum, autologous fluids such as ACP, PRP, BMA, BMC, or any combinations of these products.

The present invention also contemplates adding/binding/mixing/ compounding/incorporating SOD to/with/into degradable implants such as PLA and/or composite implants. SOD (particularly, SOD3) could be mixed with the polymer or ceramic (i.e., the implant material that will form the implant/fixation device) to obtain a mixed SOD/implant material, which is then molded or extruded/machined into different surgical implants and surgical fixation devices such as anchors, screws or pins, among many others. The SOD could be mixed throughout the fixation device (implant) offering *in vivo* exposure at implantation and throughout the degradation profile.

The SOD may be incorporated within the implant material by being mixed throughout the implant material or by being diffused throughout the implant material (for example, by melting or diffusion). The incorporation of SOD may be conducted in a homogenous or heterogenous manner, i.e., the SOD may be homogenously or heterogeneously incorporated/mixed with the implant material. The resulting implant may consist essentially of the implant material (for example, polymer, ceramic or composite material) and SOD. The SOD may be incorporated within the implant material to obtain a resulting implant with a uniform concentration profile throughout the whole body of the implant, or throughout only parts of the implant, as desired.

In additional embodiments, the SOD may be simply provided as a formulation that is coated onto the formed implant (for example, applied as a coat over the implant, during or after the manufacturing of the implant) to obtain a fully coated implant or a partially-coated implant. Either way, SOD is attached to the surgical implant and aids in the treatment of osteoarthritic tissue.

In exemplary-only embodiments, the implants are biodegradable implants used in orthopedic applications as they are associated with very low local inflammatory responses. Polymer degradation that occurs too quickly may decrease the local pH at the surgical repair site, thereby increasing the activity of osteoclasts to resorb tissue and screw material, weaken the interface, and induce inflammation. Biodegradable implants manufactured by Arthrex, Inc. are Bio (100% polymer) and BioComposite (polymer and ceramic) implants. The present invention provides improvements upon existing materials as it lessens the reaction rate when degradable anchors/implants are used.

In exemplary-only embodiments, the implants are non-biodegradable implants manufactured by Arthrex, Inc. Particular emboduments include PEEK (polyether ether ketone) and metal implants. The risk of inflammatory response or reaction is very low for both the biodegradable and non-degradable implants manufactured by Arthrex, Inc.

The molecules of the injectable solutions/formulations of the present invention may be bound covalently or by creating amine and carboxylic acid groups for binding molecules, such as with carbodimide, or some derivative of it, the most common being 1-ethyl-3-(3-dimethylaminopropyl) carbodiimide hydrochloride, and/or hydroxysuccinimide, or some derivative of it, the most common being N-hydroxysuccinimide, or by any other known binding process in the art.

In exemplary-only embodiments, molecules of the injectable solutions/formulations of the present invention may be bound as follows:
- Adding heparin or collagen to surface of or within implant or lubricating molecule, either covalently or via spacer, to bind SOD, which contains heparin and collagen-binding groups
- Binding SOD molecules via biotinylation
   ∘ Adding biotin to surface of or within implant or lubricating molecule, then bind streptavidin
   ∘ Biotinylated SOD (attaching biotin to SOD) will attach to streptavidin and provide increased binding over covalent bonding.

In additional embodiments, combination products or injectable formulations with additional oxidants such as Vitamin E (for example, natural Vitamin E or Vitamin E in natural form) are also contemplated by the present invention:
- An antioxidant is attached to a lubricating molecule or polymer protecting the lubrication molecule/polymer from being broken down from free radicals that are present within the joint. The antioxidant can be SOD or Vitamin E. The formulations may be in injectable form for direct application to intra-articular spaces and/or for implant coating or incorporation. The formulations may be provided, however, in additional forms, not limited to injectable solutions/compositions.
- An antioxidant can be combined with an autologous blood therapy such as PRP or BMA or BMC as an intra-articular injection (for direct injection). The antioxidant will block the catabolic signals that inevitably result from an autologous blood therapy allowing for the catabolic signals to become more pronounced. The antioxidant specifically is combined to not only address radicals that are already in the joint but to also address the catabolic nature that can be released by white blood cells and platelets from an autologous blood therapy. The antioxidant can be SOD or Vitamin E.

According to these embodiments, the antioxidant specifically addresses and dampens the catabolic cytokines that are within PRP therapies to enhance the overall effect of the therapy.

Vitamin E may be employed for intra-articular injection as a stand-alone, and/or with the individual components/materials listed above and as follows:
1. Intra-articular injection with Vitamin E;
2. Intra-articular injection with Vitamin E and a lubrication molecule such as HA or lubricin or a lubricating polymer or any combination of the previously-mentioned applications;
3. Intra-articular injection with Vitamin E and an anti-oxidant such as superoxide dismutase;
4. Intra-articular injection with Vitamin E and a combination of a lubrication molecule and an anti-oxidant; and
5. Intra-articular injection with Vitamin E with an autologous blood solution such as whole blood, plasma, PRP, BMA, BMC, adipose, etc.

Vitamin E by itself and/or with at least one of the components listed above (or any combinations thereof) may be injected directly into a joint, for example, directly into a patient's knee to address osteoarthritic conditions and/or intra-articular joint degeneration.

According to yet another embodiment of the present invention, Vitamin E may be combined with bone void fillers and/or biopolymers such as PLLA, PLDLA, and biocomposite, among many others. Like in the previously-described embodiments, Vitamin E (like SOD3) may be added, binded, mixed or compounded with degradable implants such as PLA and/or composite implants. Vitamin E could be mixed with the polymer or ceramic, then the resulting mixture could be molded or extruded/machined into different fixation devices and surgical implants such as anchors, screws or pins, among many others. Vitamin E could be mixed homogeneously or heterogeneously throughout the fixation device (implant) offering *in vivo* exposure at implantation and throughout the degradation profile.

The present invention provides various formulations that cover SOD (such as SOD3) and lubricin, and methods of using such formulations for intra-articular therapy and implant coating. The formulations may be employed as part of direct therapy procedures, for example, by directly injecting the formulation at the repair site, i.e., affected tissue such as arthritic tissue in the intra-articular space of a damaged joint. In exemplary-only applications and as noted above, an antioxidant (superoxide dismutase 3, SOD3 or Vitamin E) and a lubricant (lubricin and/or HA) are used as an injection therapy for osteoarthritis (OA).

The formulation(s) may be also used to coat implants or to be integrally formed with the implants, i.e., to not only help with lubrication but to also neutralize free radicals that are within the joint that can lead to poly breakdown. As SOD3 and lubricin concentrations are both reduced in joint fluid after a meniscal tear, ACL tear, and within OA joints, the inventors discovered that lubricin in conjunction with SOD, for example, can be employed as an intra-articular therapy. The lubrication product may be employed alone, or may be combined with an antioxidant in order to have a next generation product line. The combined product may optionally include natural Vitamin E, bone fillers, autologous or allogenic blood products such as plasma, autologous conditioned plasma, platelet-rich plasma, lyophilized platelets, bone marrow, bone marrow aspirate, bone marrow concentrate, stem cells such as concentrated or expanded stem cells (derived from a variety of sources), or any combinations of these products.

While the present invention is described herein with reference to illustrative embodiments for particular applications, it should be understood that the invention is not limited thereto. Those having ordinary skill in the art and access to the teachings provided herein will recognize additional modifications, applications, embodiments and substitution of equivalents all falling within the scope of the invention.

## Claims

1. A formulation for use in intra-articular space or for attachment to an implant, the formulation comprising an antioxidant and a lubricant.

2. The formulation of claim 1, wherein the antioxidant is superoxide dismutase or vitamin E.

3. The formulation of claim 1, wherein the lubricant is at least one of lubricin and hyaluronic acid.

4. A surgical articular implant comprising a formulation of any of claims 1 to 3, wherein the formulation coats the implant.

5. A surgical articular implant comprising a formulation of any of claims 1 to 4, wherein the formulation is mixed with an implant material and then machined into the implant.

6. A surgical implant consisting essentially of an implant material and superoxide dismutase.

7. The surgical implant of claim 6, wherein the superoxide dismutase is extracellular superoxide dismutase.

8. The surgical implant of claim 6, wherein the superoxide dismutase is homogenously mixed with the implant material.

9. The surgical implant of claim 6, wherein the implant material comprises UHMWPE, PLA, PGA, a composite material, or a ceramic/polymer material.

10. The surgical implant of claim 6, wherein the surgical implant is an anchor, a screw or a pin.

11. A method of forming a surgical implant, the method comprising the steps of:
providing a superoxide dismutase/implant material consisting of superoxide dismutase and an implant material; and
forming a surgical implant from the superoxide dismutase/implant material.

12. The method of claim 11, wherein the implant material is a polymer, ceramic or composite material.

13. The method of claim 11, wherein the step of forming the surgical implant further comprises molding or machining the superoxide dismutase/implant material to form the surgical implant.

14. The method of claim 11, wherein the step of providing the superoxide dismutase/implant material further comprises mixing superoxide dismutase throughout the implant material.

15. The method of claim 11, wherein the step of providing the superoxide dismutase/implant material further comprises biding superoxide dismutase to the implant material.
